(19) Europäisches Patentamt
European Patent Office
Office européen des brevets

(11) EP 0 533 518 B1

(12) **FASCICULE DE BREVET EUROPEEN**

(45) Date de publication et mention de la délivrance du brevet: **21.04.1999 Bulletin 1999/16**

(51) Int. Cl.$^6$: **A61K 31/05**, A61K 9/127, A61K 7/48, A61K 7/00

(21) Numéro de dépôt: **92402339.3**

(22) Date de dépôt: **25.08.1992**

(54) **Composition pharmaceutique pour application topique contenant du dithranol et procédé de préparation**

Dithranol enthaltendes Arzneimittel zur topischen Anwendung sowie Herstellungsverfahren

Pharmaceutical composition for topical application containing dithranol and process for its preparation

(84) Etats contractants désignés:
**AT BE CH DE DK ES GB GR IT LI PT SE**

(30) Priorité: **17.09.1991 FR 9111417**

(43) Date de publication de la demande:
**24.03.1993 Bulletin 1993/12**

(73) Titulaire: **L'OREAL**
**75008 Paris (FR)**

(72) Inventeurs:
- **Laugier, Jean-Pierre**
**F-92160 Antony (FR)**
- **Segot, Evelyne**
**F-94130 Nogent-Sur-Marne (FR)**

(74) Mandataire: **Peuscet, Jacques**
**SCP Cabinet Peuscet et Autres,**
**78, avenue Raymond Poincaré**
**75116 Paris (FR)**

(56) Documents cités:
**EP-A- 0 224 457**
**EP-A- 0 224 837**
**EP-A- 0 234 328**
**FR-A- 2 532 191**
**GB-A- 2 107 587**

- **CHEMICAL ABSTRACTS, vol. 115, no. 26, 30 Décembre 1991, Columbus, Ohio, US; abstract no. 287091g, G. MAHRLE ET AL. 'stability of anthralin in liposomal phospholipids' page 460 ;colonne 2 ;**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7/3.6

## Description

La présente invention concerne une composition pharmaceutique pour application topique contenant du dithranol et un procédé de préparation de ladite composition.

Le dithranol est un composé de formule $C_{14}H_{10}O_3$, connu également sous le nom d'anthraline, de 1,8,9 - trihydroxyanthracène ou de 1,8 - dihydroxyanthranol.

Des compositions pharmaceutiques contenant du dithranol, sont couramment utilisées pour le traitement du psoriasis par application topique sur la peau. Cependant, l'utilisation de dithranol présente de nombreux inconvénients car il a une forte action irritante pour la peau et qu'il est instable vis à vis de l'oxygéne et d'autres composés tels que l'eau et les bases alcalines. Par oxydation, il se forme à partir du dithranol une 1,8-dihydroxyanthraquinone qui elle même conduit à d'autres composés dénommés "brown anthraline" qui ont une couleur brune et qui tachent de façon indélébile les vêtements.

Pour éviter les problèmes dus à l'instabilité du dithranol, on a proposé de préparer des compositions anhydres car dans ces compositions la stabilité chimique du dithranol pose relativement moins de problème qu'en milieu aqueux. Mais ces compositions ont l'inconvénient de ne pas pouvoir être éliminées facilement à l'eau ; le temps de contact de la peau avec la composition est donc difficilement contrôlable et, par conséquent, la peau peut être irritée.

Il est connu que les compositions dans lesquelles le dithranol est dispersé dans une émulsion de type huile dans l'eau ou eau dans l'huile peuvent être éliminées par lavage à l'eau. Ces compositions permettent donc de contrôler le temps de contact du dithranol avec la peau et, par conséquent, d'effectuer le traitement des zones atteintes par le psoriasis par le procédé dit "Short Contact Therapy".

Mais, dans ces compositions contenant une phase aqueuse, il est nécessaire d'ajouter un antioxydant pour éviter l'oxydation du dithranol, oxydation qui a pour conséquence de colorer en marron la composition.

Or, il est bien connu que les antioxydants sont susceptibles de provoquer des dermatites ou des réactions allergiques de la peau (voir par exemple CONTACT DERMATITIS 1987 Vol 17 p 294-298 ; 1984 Vol 11, p 265-267 ; 1987, Vol 16 p 260-262 et 1988 Vol 5 p 313-314, MARTINDALE 29ème Ed p 1361 et 1363 ; JAAD Vol 13 N6 Déc 1985 p 1062-1069 (ADAMS R et RAIBACH H) ; JID 85 : 351-356, 1985 (DENEZZA et al) et DE-A 3 540 175).

On cherche par conséquent à obtenir une composition dans laquelle le dithranol soit stable en phase aqueuse sans qu'il soit nécessaire d'ajouter un antioxydant.

Dans DE-A 3 540175, on a proposé de stabiliser les compositions contenant du dithranol par addition d'un additif auto-émulsifiant qui peut être un ester de glycéride d'acide gras, un ester d'acide gras de l'acide lactyllactique, un ester d'acide gras polyglycérolé ou des esters 2 - hydroxyalkylés. Des compositions ont été préparées selon les exemples 2 et 3 de cette demande et ont été laissées en contact avec l'air. On a observé, au bout de quelques heures, la formation d'une coloration brune due à l'oxydation du dithranol.

Il est également connu par DE-A 3 542 773 que l'on améliore le taux de pénétration des principes actifs, notamment du dithranol, en encapsulant ces principes actifs dans des liposomes préparés à partir de lipides amphiphiles ioniques tels que les phospholipides. Mais il faut noter que, comme montré dans les exemples comparatifs donnés ci-après, l'encapsulation du dithranol dans des liposomes n'empêche pas l'oxydation de celui-ci.

Selon la présente invention, on a trouvé de façon inattendue qu'en ajoutant à une émulsion de type huile dans l'eau contenant du dithranol, des vésicules contenant un lipide amphiphile non-ionique, on empêche l'oxydation du dithranol.

La présente invention a donc pour objet une composition pharmaceutique pour application topique comportant une émulsion de type huile dans l'eau contenant du dithranol, caractérisé par le fait que l'émulsion contient sous forme dispersée des vésicules à partir d'une phase lipidique contenant au moins un lipide amphiphile non-ionique.

En effet, on a trouvé qu'en présence des vésicules de phase lipidique contenant un lipide amphiphile non-ionique ou niosome, le dithranol contenu dans une émulsion huile dans l'eau ne s'oxyde pas. Le dithranol est donc stable et la teneur en dithranol des compositions ne décroît pas au cours du stockage. De plus, les compositions contenant des vésicules non-ioniques ou niosomes ne se colorent pas ou ne se colorent que faiblement en marron lorsqu'elles sont en contact avec l'air. L'utilisateur ne risque donc pas de tacher de façon indélébile ses vêtements.

Par ailleurs, il faut noter que de façon connue, comme décrit dans FR-A 2 490 504, les vésicules non-ioniques, stabilisent l'émulsion huile dans l'eau contenant le dithranol. Il n'est donc pas nécessaire d'ajouter un agent émulsifiant.

La composition selon l'invention est donc constituée par une phase aqueuse dans laquelle sont dispersées les gouttelettes d'huile de l'émulsion huile dans l'eau et les vésicules non-ioniques. Le dithranol se trouve à l'état solubilisé ou dispersé dans l'huile ou dans la phase aqueuse. Il faut noter qu'il n'est pas encapsulé dans les vésicules non-ioniques.

Les lipides amphiphiles non-ioniques contenus dans la phase lipidique sont, de préférence, ceux ayant pour formule :

$$R_1O-\left[C_3H_5(OH)O\right]_{\bar{n}}-H \qquad (I)$$

formule dans laquelle :

. $-C_3H_5(OH)O$ - est représenté par les structures suivantes prises en mélange ou séparément : $-CH_2-CH(OH)-CH_2O-$ ;

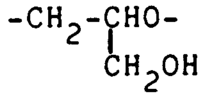 ; 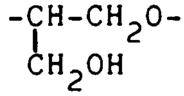 ;

. $\bar{n}$ est une valeur statistique moyenne comprise entre 2 et 6 ;
. $R_1$ représente un reste de formule : où :
. $R_2$ représente un radical alkyle, linéaire ou ramifié, saturé ou insaturé, contenant de 12 à 18 atomes de carbone, ou bien un reste $R_4CO$ où $R_4$ est un radical aliphatique, linéaire ou ramifié, en $C_{11}$ - $C_{17}$ ;
. $-OC_2H_3(R_3)-$ est représenté par les structures suivantes, prises en mélange ou séparément :

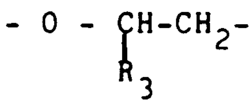 et 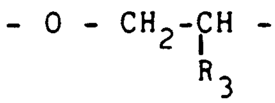

où $R_3$ prend la signification donnée par $R_2$.

De façon connue on peut associer, dans la phase lipidique, au(x) lipide(s) amphiphile(s) non-ionique(s) au moins un additif destiné à améliorer la stabilité physicochimique et la perméabilité aux substances encapsulées des vésicules. Parmi les additifs connus, on peut citer les alcools et les diols à longue chaîne ; les stérols, plus particulièrement le cholestérol et le béta-sitostérol ; les amines à longue chaîne et leurs dérivés ammonium quaternaires, par exemple le bromure de dodécyldiméthylammonium ou des bishydroxyalkylamines ; des amines grasses polyoxyéthylénées ou leurs sels ; des esters d'aminoalcools à longue chaîne ainsi que leurs sels et dérivés ammonium quaternaires ; des alkylsulfates, par exemple le cétylsulfate de sodium ; et des dérivés ioniques des stérols, tels que les phosphate et sulfate de cholestérol. Dans le cadre de la présente invention, les esters phosphoriques d'alcool gras, par exemple le dicétyl ou le dimyristylphosphate de sodium, sont particulièrement appropriés ; ces esters phosphoriques peuvent avantageusement être également associés à du cholestérol.

Les vésicules non-ioniques ou niosomes peuvent de façon connue encapsuler des substances hydrophiles et/ou lipophiles ayant une activité cosmétique ou dermopharmaceutique.

La phase lipidique sous forme de vésicules non-ioniques représente entre 2 % et 14 % en poids de la composition.

La composition pharmaceutique contient de 0,05 à 5 % en poids de dithranol par rapport à la composition, de préférence 0,2 à 2 % en poids, le dithranol est introduit sous forme de poudre, de préférence micronisée lorsque sa concentration est supérieure à 0,15 % en poids.

La phase huileuse de l'émulsion de type huile dans l'eau est, de manière générale, constituée par une huile saturée liquide à température ambiante. Cette huile peut être une huile d'origine végétale ou animale prise dans le groupe formé par les esters d'acides gras et de polyols, tels que le tricaprocaprylate de glycérol et les esters d'acide gras et d'alcools ramifiés de formule $R_5CO\ O\ R_6$ dans laquelle $R_5$ représente le reste d'un acide gras supérieur en $C_8$ - $C_{20}$ et $R_6$ représente une chaîne hydrocarbonée en $C_3$ - $C_{20}$ ,tels que le myristate d'isopropyle.

L'huile peut également être un hydrocarbure ou un polysiloxane. L'hydrocarbure est, par exemple, la vaseline, l'huile de paraffine, ou le perhydrosqualène. Le polysiloxane est, par exemple, la cyclométhicone, le diméthylpolysiloxane ou le phénylpolysiloxane.

La quantité d'huile présente dans la composition est comprise entre 5 et 30 % en poids de la composition, de préférence entre 15 et 25 %.

De façon connue, des additifs cosmétiques ou dermopharmaceutiques liposolubles peuvent être introduits

dans la phase huileuse.

La phase aqueuse de l'émulsion peut également contenir des actifs cosmétiques ou dermopharmaceutiques hydrosolubles.

Selon la présente invention, la phase aqueuse est de préférence gélifiée. Parmi les gélifiants utilisables, on peut citer les dérivés de cellulose, des dérivés d'algues ou des gommes naturelles. On préfère utiliser l'hydroxyéthylcellulose ou un acide polycarboxyvinylique tel que, par exemple, le "CARBOPOL 940" vendu par la société GOODRICH.

L'agent gélifiant est ajouté, en général, en quantités comprises entre 0,1 et 2 % en poids par rapport à la composition.

On peut également, de façon connue, introduire dans la composition selon l'invention des additifs pharmaceutiquement acceptables, n'ayant pas d'activité pharmaceutique, généralement utilisés pour la formulation de compositions pour application topique. Ces additifs pharmaceutiquement acceptables sont, de préférence, pris dans le groupe formé par les bactéricides, les conservateurs, les agents chélatants, les opacifiants, ou les colorants ; on ajoute en particulier des conservateurs antibactériens tels que le parahydroxybenzoate de méthyle et le parahydroxybenzoate de propyle.

La composition est, de préférence, préparée de la façon suivante :

- on prépare par tout procédé connu à partir d'une phase lipidique des vésicules sous forme de dispersion dans une phase aqueuse ;
- on prépare séparément par ailleurs une dispersion de dithranol en poudre dans la phase huileuse ;
- éventuellement, on prépare également séparément un gel d'un agent gélifiant dans une phase aqueuse ;
- on incorpore dans un homogénéiseur, la dispersion de dithranol dans la phase huileuse dans la dispersion de vésicules dans une phase aqueuse ;
- et pour finir éventuellement, on ajoute le gel.

De préférence, la dispersion de vésicules dans une phase aqueuse est réalisée par fusion de la phase lipidique, hydratation de la phase lipidique fondue sous agitation de façon à obtenir une phase lamellaire puis addition d'une quantité supplémentaire de phase aqueuse et homogénéisation de façon à obtenir des vésicules sous forme de dispersion dans une phase aqueuse.

Les exemples donnés ci-après sont donnés pour illustrer l'invention sans la limiter.

<u>EXEMPLE 1 (COMPARATIF)</u>

On a préparé à titre comparatif 3 compositions contenant du dithranol dans une émulsion de type huile dans l'eau.

A) Composition A selon l'invention ayant la formulation suivante (en % en poids) :

- Phase lipidique............................ 8 %

  composée de :

  . Lipide amphiphile non-ionique constitué par de l'hexadécyléther de diglycérol de formule II

  . $C_{16}H_{33}O\ (CH_2 - \underset{OH}{\underset{|}{CH}} - CH_2O)_2H$ ..... 3,8 %

  . Dicéthyl phosphate............... 0,4 %

  . Cholestérol...................... 3,8 %

- Triglycéride d'acides caprylique et caprique vendu sous la dénomination commerciale "MIGLYOL 812" par la société DYNAMIT NOBEL. 15 %
- Dithranol.................................. 0,3 %
- Acide polycarboxyvinylique vendu sous la dénomination commerciale "CARBOPOL 940" par la société GOODRICH........................ 0,6 %
- Triéthanolamine.....................qsp pH 3,6
- Eau purifiée.........................qsp... 100,0 %

La composition A est préparée de la façon suivante. On réalise la fusion à 110°C de la phase lipidique, puis on hydrate la phase lipidique fondue à l'aide de 22 % d'eau en poids par rapport à la composition totale à 80°C pour obtenir une phase lamellaire ; on poursuit l'hydratation par addition de 30 % d'eau en poids par rapport à la composition totale à 70°C et on passe à l'homogénéiseur haute pression pour obtenir des vésicules dispersées dans l'eau.

Par ailleurs, on prépare une suspension de dithranol dans l'huile constituée par le "MIGLYOL 812" à l'aide d'une turbine.

On prépare, à part, un gel de "CARBOPOL 940" neutralisé par la triéthanolamine.

Pour finir, on incorpore la suspension de dithranol dans la dispersion de vésicules dans l'eau, à l'aide de l'homogénéiseur haute pression puis on incorpore le gel de "CARBOPOL 940" neutralisé.

B) Composition B (ne faisant pas partie de l'invention) contenant du dithranol dans une émulsion de type huile dans l'eau avec un tensioactif anionique.

La composition a la formulation suivante (en % en poids) :

| | |
|---|---|
| - Lauryl sulfate de sodium | 8 % |
| - "MIGLYOL 812" | 15 % |
| - Dithranol | 0,30 % |
| - "CARBOPOL 940" | 0,60 % |
| - Triéthanolamine qsp PH | 3,6 |
| - Eau qsp | 100,0 % |

C) Composition C (ne faisant pas partie de l'invention) contenant du dithranol dans une émulsion huile dans l'eau avec un tensioactif non-ionique.

La composition a la formulation suivante :

- Tensioactif non-ionique.................... 8 %
  constitué de :
  . un mélange de glycérylstéarate et de stéarate de polyéthylène glycol 100 vendu sous la dénomination commerciale "ARLACEL 165" par la société ICI.............. 6 %
  . un polysorbate vendu sous la dénomination commerciale "TWEEN 60" par la société ICI.................. 2 %
- "MIGLYOL 812"............................ 15 %
- Dithranol................................ 0,30 %
- "CARBOPOL 940"........................... 0,60 %
- Triéthanolamine......................qsp pH 3,6
- Eau purifiée........................qsp... 100,0 %

D) Composition (ne faisant pas partie de l'invention) contenant des vésicules de lipide amphiphile ionique.

| | |
|---|---|
| - Phase lipide constituée de lécithine de soja vendue sous la dénomination commerciale "EPIKURON 145 V" par la société LUCAS MEYER | 8 % |
| - "MIGLYOL 812" | 15 % |
| - Dithranol | 0,30 % |
| - "CARBOPOL 940" | 0,60 % |
| - Triéthanolamine qsp pH | 3,6 |
| - Eau purifiée qsp | 100,0 % |

Cette composition D est préparée par un procédé analogue à celui de la composition A.

On a laissé 100 g des compositions A à D en contact avec l'air à différentes températures pendant 1 et 2 mois puis on a dosé, en grammes, la quantité de dithranol restant dans la composition après exposition à l'air, et calculé le pourcentage de dégradation.

Les résultats sont donnés dans le tableau I ci-après.

| COMPOSITION | A | | B (1) | | C (1) | | D (1) | |
|---|---|---|---|---|---|---|---|---|
| | Poids de dithranol en g | % de dégradation | Poids de dithranol en g | % de dégradation | Poids de dithranol en g | % de dégradation | Poids de dithranol en g | % de dégradation |
| **Temps 0** | **0,276** | **-** | **0,303** | **-** | **0,263** | **-** | **0,239** | **-** |
| **1 mois T.A.** (2) | **0,273** | **1,08** | **0,259** | **14** | **0,255** | **3** | **0,229** | **4** |
| **2 mois T.A.** | **0,278** | **NS** (3) | **0,248** | **18** | **0,243** | **7,6** | **0,255** | **6** |
| **1 mois 37°C** | **0,273** | **1,08** | **0,251** | **17** | **0,248** | **5,7** | **0,227** | **7** |
| **2 mois 37°C** | **0,274** | **NS** (3) | **0,220** | **27** | **0,220** | **16** | **0,216** | **9,6** |
| **1 mois 45°C** | **0,274** | **NS** (3) | **0,237** | **21,7** | **0,212** | **19** | **0,221** | **7,5** |
| **2 mois 45°C** | **0,274** | **NS** (3) | **0,111** | **63** | **0,118** | **55** | **0,121** | **49** |

(1) Ne fait pas partie de l'invention.
(2) T.A. : Température ambiante.
(3) NS : non significatif.

Le tableau I montre que le dithranol n'est stable que dans la composition A selon l'invention. Dans les autres compositions (B, C, D), il serait nécessaire d'ajouter un antioxydant pour stabiliser le dithranol.

EXEMPLE 2 : CREME A 2,5 % DE DITHRANOL

On a préparé par un procédé analogue à celui décrit pour la composition A, une crème ayant la formulation suivante :

**- lipide amphiphile non-ionique de formule :**

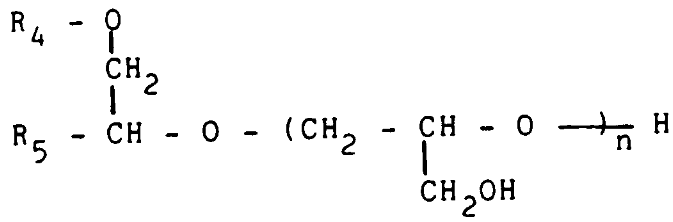

**n = 6**

**$R_4 = C_{12}H_{25}$**

**$R_5 = C_{14}H_{29}/C_{16}H_{33}$**............................ **10,0 g**

**- Ditétradécylphosphate**........................ **1,0 g**

**- Perhydrosqualène**........................... **25,0 g**

**- Dithranol micronisé**........................ **2,5 g**

**- Acide polycarboxyvinylique vendu sous la dénomination commerciale "CARBOPOL 940" par la société GOODRICH**.................... **0,6 g**

**- Parahydroxybenzoate de méthyle**............. **0,1 g**

**- Parahydroxybenzoate de propyle**............. **0,05 g**

**- Soude à 10 %**.........................**qs pH.. 3,6**

**- Eau purifiée**.........................**qsp.... 100,0 g**

On a vérifié qu'après 48 heures d'expositions à l'air à température ambiante, la crème n'a pas pris une coloration brune.

<u>EXEMPLE 3 : CREME A 2 % DE DITHRANOL</u>

On a préparé une crème ayant la formulation suivante :

| | |
|---|---|
| - Lipide amphiphile non-ionique de l'exemple 1 | 9,0 g |
| - Ditétradécylphosphate | 1,0 9 |
| - Huile de vaseline | 25,0 g |
| - Dithranol micronisé | 2,0 g |
| - Acide polycarboxyvinylique vendu sous la dénomination "CARBOPOL 940" par la société GOODRICH | 0,75 g |
| - Parahydroxybenzoate de méthyle | 0,1 g |
| - Parahydroxybenzoate de propyle | 0,05 9 |
| - Triéthanolamine qs pH. | 3,5 |
| - Eau purifiée qsp | 100,0 g |

On a vérifié qu'après 48 heures d'exposition à l'air à température ambiante la crème n'a pas pris une coloration brune.

EXEMPLE 4 : CREME A 2 % DE DITHRANOL

On a préparé une crème ayant la formulation suivante :

| | |
|---|---|
| - Lipide amphiphile non-ionique de l'exemple 1 | 9,0 9 |
| - Ditétradécylphosphate | 1,0 g |
| - Huile de vaseline | 25,0 9 |
| - Dithranol micronisé | 2,0 9 |
| - Acide polycarboxyvinylique vendu sous la dénomination "CARBOPOL 940" par la société GOODRICH | 0,75 9 |
| - Parahydroxybenzoate de méthyle | 0,1 g |
| - Parahydroxybenzoate de propyle | 0,05 9 |
| - Triéthanolamine qs pH | 3,5 |
| - Eau purifiée qsp | 100,0 9 |

On a vérifié qu'après 48 heures d'exposition à l'air à température ambiante la crème n'a pas pris une coloration brune.

EXEMPLE 5 : CREME A 0,05 % DE DITHRANOL

On a préparé une crème ayant la formulation suivante :

- Lipide amphiphile non-ionique de formule :

$$R_6 - O - (CH_2 - \underset{\displaystyle CH_2OH}{\underset{|}{CH}} - O)_n - H$$

n = 3

$R_6 = C_{16}H_{33}$.................................. 3,8 g
- Cholestérol.................................. 3,8 g
- Phosphate de dihéxadécyle.................. 0,4 g
- Triglycéride d'acides caprylique et caprique vendu sous la dénomination "MIGLYOL 812" par la société DYNAMIT NOBEL............... 15,0 g
- Dithranol.................................. 0,05 g
- Acide polycarboxyvinylique vendu sous la dénomination "CARBOPOL 940" par la société GOODRICH.................................. 0,6 g
- Parahydroxybenzoate de méthyle............. 0,1 g
- Parahydroxybenzoate de propyle............. 0,05 g
- Triéthanolamine.....................qs pH.. 3,4
- Eau purifiée........................qsp.... 100,0 g

On a vérifié qu'après 48 heures d'exposition à l'air à température ambiante la crème n'a pas pris une coloration brune.

## Revendications

1. Composition pharmaceutique pour application topique comportant une émulsion de type huile dans l'eau contenant du dithranol, caractérisée par le fait que l'émulsion contient sous forme dispersée des vésicules préparées à partir d'une phase lipidique contenant au moins un lipide amphiphile non-ionique.

2. Composition selon la revendication 1, caractérisée par le fait que le(s) lipide(s) amphiphile(s) non-ionique(s) a(ont) pour formule :

$$R_1 O - [C_3H_5(OH)O]_{\bar{n}} - H$$

formule dans laquelle :

. $-C_3H_5(OH)O-$ est représenté par les structures suivantes prises en mélange ou séparément : $-CH_2-CH(OH)-CH_2O-$ ;

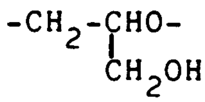 ; 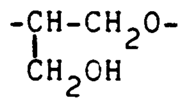 ;

. $\bar{n}$ est une valeur statistique moyenne comprise entre 2 et 6 ;
. $R_1$ représente un reste de formule : $R_2\{OC_2H_3(R_3)\}$ où :
. $R_2$ représente un radical alkyle, linéaire ou ramifié, saturé ou insaturé, contenant de 12 à 18 atomes de carbone, ou bien un reste $R_4$ CO où $R_4$ est un radical aliphatique, linéaire ou ramifié, en $C_{11}$ - $C_{17}$ ;
. $-OC_2H_3$ ($R_3$) - est représenté par les structures suivantes, prises en mélange ou séparément :

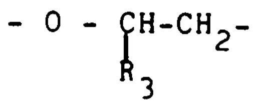 et 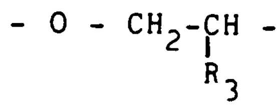

où $R_3$ prend la signification donnée par $R_2$.

3. Composition selon l'une des revendications 1 ou 2, caractérisée par le fait qu'au moins un additif destine à améliorer la stabilité et la stabilité des vésicules est associe dans la phase lipide au(x) lipide(s) amphiphile(s) non-ionique(s).

4. Composition selon l'une des revendications 1 à 3, caractérisée par le fait que les vésicules non-ioniques encapsulent des substances hydrophiles et/ou lipophiles ayant une activité cosmétique ou dermopharmaceutique.

5. Composition selon l'une des revendications 1 à 4, caractérisée par le fait que la phase lipidique sous forme de vésicules non-ioniques représente entre 2 % et 14 % en poids de la composition.

6. Composition selon l'une des revendications 1 à 5, caractérisée par le fait qu'elle contient de 0,05 à 5 % en poids de dithranol.

7. Composition selon l'une des revendications 1 à 6, caractérisée par le fait qu'elle contient de 0,2 à 2 % en poids de dithranol.

8. Composition selon l'une des revendications 1 à 7, caractérisée par le fait que la phase huileuse de l'émulsion huile dans l'eau est une huile d'origine végétale ou animale, un hydrocarbure ou un polysiloxane.

9. Compositions selon l'une des revendications 1 à 8, caractérisée par le fait que la phase huileuse contient un actif cosmétique ou dermopharmaceutique oléosoluble.

10. Composition selon l'une des revendications 1 à 9, caractérisée par le fait que la phase aqueuse de l'émulsion de type huile dans l'eau contient un actif cosmétique ou dermopharmaceutique hydrosoluble.

11. Composition selon l'une des revendications 1 à 10, caractérisée par le fait que la phase aqueuse de l'émulsion huile dans l'eau est gélifiée par un agent gélifiant.

12. Composition selon la revendication 11, caractérisée par le fait que l'agent gélifiant est un acide polycarboxyvinylique.

13. Compositions selon l'une des revendications 11 ou 12, caractérisée par le fait que l'agent gélifiant représente 0,1 à 2 % en poids de la composition.

14. Procédé de préparation de la composition selon l'une des revendications 1 à 13, caractérisé par le fait que :

- on prépare à partir de la phase lipidique des vésicules sous forme de dispersion dans une phase aqueuse ;
- on prépare séparément par ailleurs une dispersion de dithranol en poudre dans la phase huileuse et ;
- on incorpore dans un homogénéiseur la dispersion de dithranol dans la phase huileuse dans la dispersion de vésicules non-ioniques dans la phase aqueuse.

**15.** Procédé selon la revendication 14, caractérisé par le fait qu'on prépare séparément un gel d'un agent gélifiant dans une phase aqueuse et on l'ajoute à la composition après incorporation de la dispersion de dithranol dans la dispersion de vésicules.

## Claims

**1.** Pharmaceutical composition for topical application comprising an oil-in-water type emulsion containing dithranol, characterised in that the emulsion contains, in dispersed form, vesicles prepared from a lipid phase containing at least one nonionic amphiphilic lipid.

**2.** Composition according to claim 1, characterised in that the nonionic amphiphilic lipid(s) is(are) of formula:

$$R_1O-[C_3H_5(OH)O]_{\bar{n}}-H$$

formula in which:

. $-C_3H_5(OH)-O-$ is represented by the following structures taken together or separately: $-CH_2-CH(OH)-CH_2O-$;

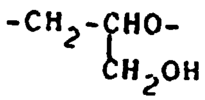 ; 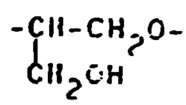 ;

. $\bar{n}$ is a mean statistical value between 2 and 6;
. $R_1$ represents a residue of formula: $R_2[OC_2H_3(R_3)]$ where:
. $R_2$ represents a linear or branched, saturated or unsaturated alkyl radical containing 12 to 18 carbon atoms or, alternatively, a $R_4CO$ residue where $R_4$ is a linear or branched $C_{11}$-$C_{17}$ aliphatic radical;
. $OC_2H_3(R_3)$- is represented by the following structures taken together or separately:

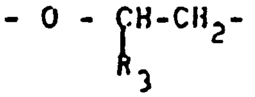 and 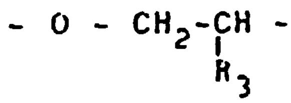

where $R_3$ has the meaning given for $R_2$.

**3.** Composition according to one of claims 1 or 2, characterised in that at least one additive intended to improve the stability and permeability of the vesicles is combined, in the lipid phase, with the nonionic amphiphilic lipid(s).

**4.** Composition according to one of claims 1 to 3, characterised in that the nonionic vesicles encapsulate hydrophilic and/or lipophilic substances having a cosmetic or dermopharmaceutical activity.

**5.** Composition according to one of claims 1 to 4, characterised in that the lipid phase in the form of nonionic vesicles represents between 2 % and 14 % by weight of the composition.

**6.** Composition according to one of claims 1 to 5, characterised in that it contains 0.05 to 5 % by weight of dithranol.

7. Composition according to one of claims 1 to 6, characterised in that it contains 0.2 to 2 % by weight of dithranol.

8. Composition according to one of claims 1 to 7, characterised in that the oily phase of the oil-in-water emulsion is an oil of vegetable or animal origin, a hydrocarbon or a polysiloxane.

9. Compositions according to one of claims 1 to 8, characterised in that the oily phase contains an oil-soluble cosmetic or dermopharmaceutical active ingredient.

10. Composition according to one of claims 1 to 9, characterised in that the aqueous phase of the oil-in-water type emulsion contains a water-soluble cosmetic or dermopharmaceutical active ingredient.

11. Composition according to one of claims 1 to 10, characterised in that the aqueous phase of the oil-in-water emulsion is gelled by a gelling agent.

12. Composition according to claim 11, characterised in that the gelling agent is a polycarboxyvinylic acid.

13. Compositions according to one of claims 11 or 12, characterised in that the gelling agent represents 0.1 to 2 % by weight of the composition.

14. Process for preparing the composition according to one of claims 1 to 13, characterised in that:

- vesicles in the form of a dispersion in an aqueous phase are prepared from a lipid phase;
- in addition, a dispersion of powdered dithranol in the oily phase is prepared separately;
- the dispersion of dithranol in the oily phase is incorporated, in a homogeniser, into the dispersion of nonionic vesicles in an aqueous phase.

15. Process according to claim 14, characterised in that a gel is prepared separately from a gelling agent in an aqueous phase and it is added to the composition after incorporating the dispersion of dithranol into the dispersion of vesicles.

**Patentansprüche**

1. Pharmazeutische Zusammensetzung zur topischen Anwendung, die eine Dithranol enthaltende Öl-in-Wasser-Emulsion enthält, dadurch gekennzeichnet, daß die Emulsion in dispergierter Form Bläschen enthält, die aus einer mindestens ein amphiphiles nichtionisches Lipid enthaltenden Lipidphase hergestellt sind.

2. Zusammensetzung nach Anspruch 1, dadurch gekennzeichnet, daß das/die amphiphile/n nichtionische/n Lipid/e die Formel hat/haben:

$$R_1\,O-\left[C_3H_5(OH)O\right]_{\bar{n}}H$$

in der

. $-C_3H_5(OH)O-$ von den folgenden Strukturen in Mischung oder getrennt gebildet wird: $-CH_2-CH(OH)-CH_2O-$ ;

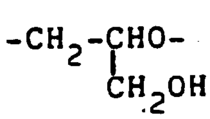 ; 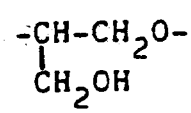 ;

. $\bar{n}$ ein statistischer Mittelwert zwischen 2 und 6 ist,
. $R_1$ einen Rest der Formel $R_2[OC_2H_3(R_3)]$ darstellt, in welcher

. $R_2$ einen verzweigten oder linearen, gesättigten oder ungesättigten Alkylrest darstellt, der 12 bis 18 Kohlenstoffatome enthält, oder einen $R_4CO$-Rest, in dem $R_4$ ein linearer oder verzweigter aliphatischer $C_{11}$-$C_{17}$-Rest ist,

. $-OC_2H_3(R_3)-$ von den folgenden Strukturen in Mischung oder einzeln gebildet wird:

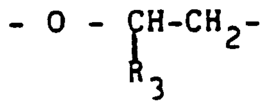 und 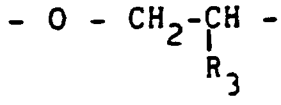

worin $R_3$ die Bedeutung von $R_2$ hat.

3. Zusammensetzung nach einem der Ansprüche 1 oder 2, dadurch gekennzeichnet, daß dem/den amphiphilen nichtionischen Lipid/en in der Lipidphase mindestens ein Zusatz beigegeben ist, der zur Verbesserung der Stabilität und der Stabilität der Bläschen bestimmt ist.

4. Zusammensetzung nach einem der Ansprüche 1 bis 3, dadurch gekennzeichnet, daß in den nichtionischen Bläschen hydrophile und/oder lipophile Substanzen mit kosmetischer oder dermopharmazeutischer Wirkung eingekapselt sind.

5. Zusammensetzung nach einem der Ansprüche 1 bis 4, dadurch gekennzeichnet, daß die Lipidphase in Form von nichtionischen Blaschen 2 bis 14 Gew.-% der Zusammensetzung darstellt.

6. Zusammensetzung nach einem der Ansprüche 1 bis 5, dadurch gekennzeichnet, daß sie 0,05 bis 5 Gew.-% Dithranol enthält.

7. Zusammensetzung nach einem der Ansprüche 1 bis 6, dadurch gekennzeichnet, daß sie 0,2 bis 2 Gew.-% Dithranol enthält.

8. Zusammensetzung nach einem der Ansprüche 1 bis 7, dadurch gekennzeichnet, daß die Ölphase der Öl-in-Wasser-Emulsion ein Öl pflanzlichen oder tierischen Ursprungs, ein Kohlenwasserstoff oder ein Polysiloxan ist.

9. Zusammensetzung nach einem der Ansprüche 1 bis 8, dadurch gekennzeichnet, daß die Ölphase einen öllöslichen kosmetischen oder dermopharmazeutischen Wirkstoff enthält.

10. Zusammensetzung nach einem der Ansprüche 1 bis 9, dadurch gekennzeichnet, daß die wässrige Phase der Öl-in-Wasser-Emulsion einen wasserlöslichen kosmetischen oder dermopharmazeutischen Wirkstoff enthält.

11. Zusammensetzung nach einem der Ansprüche 1 bis 10, dadurch gekennzeichnet, daß die wässrige Phase der Öl-in-Wasser-Emulsion durch ein Geliermittel geliert ist.

12. Zusammensetzung nach Anspruch 11, dadurch gekennzeichnet, daß das Geliermittel eine Polycarboxyvinylsäure ist.

13. Zusammensetzung nach einem der Ansprüche 11 oder 12, dadurch gekennzeichnet, daß das Geliermittel 0,1 bis 2 Gew.-% der Zusammensetzung darstellt.

14. Verfahren zur Herstellung der Zusammensetzung nach einem der Ansprüche 1 bis 13, dadurch gekennzeichnet, daß man

- aus der Lipidphase Bläschen in Form von Dispersion in einer wässrigen Phase herstellt,
- getrennt eine Dispersion von pulverförmigem Dithranol in der Ölphase herstellt und
- in einem Homogenisiergerät die Dispersion von Dithranol in der Ölphase in die Dispersion der nichtionischen Bläschen in wässriger Phase einführt.

15. Verfahren nach Anspruch 14, dadurch gekennzeichnet, daß man getrennt ein Gel eines Geliermittels in einer wässrigen Phase herstellt und es der Zusammensetzung nach Einführung der Dithranoldispersion in die Bläschen-

dispersion beigibt.